# EUROPEAN PATENT SPECIFICATION

(11) **EP 2 569 637 B1**
(45) Date of publication and mention of the grant of the patent: **08.04.2015**
(21) Application number: 11724363.4
(22) Date of filing: 09.05.2011
(51) Int. Cl.: G01N 33/574

(54) **METHODS FOR THE DIAGNOSIS AND PROGNOSIS OF A TUMOR USING BCAT1 PROTEIN**
VERFAHREN ZUR TUMORDIAGNOSE UND -PROGNOSE MITTELS BCAT1-PROTEIN
MÉTHODES POUR DIAGNOSTIQUER ET PRONOSTIQUER UNE TUMEUR À L'AIDE DE LA PROTÉINE BCAT1

(30) Priority: 14.05.2010 US 334812 P
(43) Date of publication of application: 20.03.2013
(73) Proprietor: Deutsches Krebsforschungszentrum, 69120 Heidelberg (DE)
(72) Inventor: RADLWIMMER, Bernhard, 69117 Heidelberg (DE); BARBUS, Sebastian, 69115 Heidelberg (DE); TÖNJES, Martje, 69115 Heidelberg (DE); TÖDT, Grischa, 69115 Heidelberg (DE); LICHTER, Peter, 69251 Gaiberg (DE); REIFENBERGER, Guido, 40591 Düsseldorf (DE)
(74) Representative: Schüssler, Andrea
(86) International application number: PCT/EP2011/002307
(87) International publication number: WO 2011/141153

(56) References cited:
- WO-A2-03/039443
- US-A1- 2007 128 639
- YOSHIKAWA REIGETSU ET AL: "ECA39 is a novel distant metastasis-related biomarker in colorectal cancer", WORLD JOURNAL OF GASTROENTEROLOGY, WJG PRESS, CN, vol. 12, no. 36, 28 September 2006 (2006-09-28), pages 5884-5889, XP002638238, ISSN: 1007-9327
- BELLO M ET AL: "Molecular analysis of genomic abnormalities in human gliomas", CANCER GENETICS AND CYTOGENETICS, ELSEVIER SCIENCE PUBLISHING, NEW YORK, NY, US, vol. 73, no. 2, 1 April 1994 (1994-04-01), pages 122-129, XP025212953, ISSN: 0165-4608, DOI: DOI:10.1016/0165-4608(94)90195-3 [retrieved on 1994-04-01]
- HORBINSKI CRAIG ET AL: "Diagnostic use of IDH1/2 mutation analysis in routine clinical testing of formalin-fixed, paraffin-embedded glioma tissues.", JOURNAL OF NEUROPATHOLOGY AND EXPERIMENTAL NEUROLOGY DEC 2009 LNKD- PUBMED:19915484, vol. 68, no. 12, December 2009 (2009-12), pages 1319-1325, XP009150477, ISSN: 0022-3069

## Description

The present invention relates to methods for the diagnosis of a brain tumor, and for the estimation of a prognosis for patients afflicted with such a tumor based on the determination of the expression of BCAT1 in a patient sample.

### BACKGROUND OF THE INVENTION

Cancer is the second leading cause of death in the United States after cardiovascular disease. One in three Americans will develop cancer in his or her lifetime, and one of every four Americans will die of cancer. Malignant human gliomas account for the largest number of human malignant brain tumors. So far, the treatment of gliomas includes neurosurgical techniques (resection or stereotactic procedures), radiation therapy and chemotherapy. However, despite these therapies gliomas are considered as nearly incurable as they fail to respond to ionising radiation, chemotherapy and surgical resection. In other words, with these therapies only a very limited prolongation of lifespan of patients can be achieved, i.e. despite these therapies, the average life span after diagnosis is merely 12 to 16 months. The knowledge of prognostic factors might be decisive for the selection of the preferable kind of life prolonging therapy.

Yoshikawa Reigetsu et al. (World J. of Gastroenterology, WJG Press, CN, Vol. 12, No. 36, pp. 5884-5889) describes the use of ECA 39 as marker in colorectal cancer.

### SUMMARY OF THE INVENTION

The technical problem underlying the present invention is (a) providing improved diagnostic methods for a brain tumor and (b) providing an overall survival or progression prognosis for patients having such a tumor, leading to a distinct decision of a physician for a particular kind of treatment.

The solution to said technical problem is achieved by providing the embodiments characterized in the claims.

IDH1 mutations occur in a high frequency in WHO grade II and III diffuse gliomas. 93% of all IDH1 mutations are characterized by an amino acid exchange R132H. It could be demonstrated that patients harbouring IDH1 mutations have a better prognosis compared to patients without an IDH1 mutation. Similarly, patients with IDH2 mutations also have a better prognosis in comparison to patients without an IDH2 mutation. This effect was found to be independent from other established molecular markers like losses on 1p/19q and methylation of the MGMT promoter. This observation shows that the analysis of the IDH1 or IDH2 status is of great interest in the field of neurooncology and is useful as a prognostic or predictive marker. Moreover, the knowledge of the IDH1 or IDH2 status has consequences for decisions of the attending physician regarding the particular kind of treatment of patients with (diffuse) gliomas.

The method of the present invention overcomes the problems associated with the direct determination of the mutation status of an IDH1/2 gene. An immunohistochemical assay for classifying tumors based on differences in the metabolism of branched chain amino acids was developed. In brain tumors the assay distinguishes tumors harboring mutations in either the IDH1 or IDH2 genes or both from tumors with wild type IDH1 and IDH2 genes. That way the IDH1/2 status and activity of branched chain amino acid metabolism can be determined using tissue slides. Accordingly, this approach is a fast and simple diagnostic and prognostic stratification of tumors based on branched chain amino acid metabolism activity, e.g., of the IDH1 and IDH2 mutation status by immunohistochemistry.

Moreover, the current analysis of the mutation status of the IDH1/IDH2 gene requires either DNA-sequence analysis or immunohistochemical analysis using, e.g., an IDH1-R132H antibody. Mutation analysis of the IDH2 gene requires DNA-sequence analysis. There is no immunohistochemical assay available for mutant IDH2 protein. By use of the method of the present invention results can be generated much faster and less expensive than by DNA sequencing. Unlike DNA sequencing technology, immunohistochemical analysis is routinely available in diagnostic laboratories. Due to its high sensitivity, the technique may also be used for the indirect diagnostic assessment of IDH1 or IDH2 mutation in tissue samples with low tumor cell content. Moreover, immunohistochemical analysis using an anti-BCAT1 antibody is more sensitive and more specific than immunohistochemical analysis using, e.g., the IDH1-R132H antibody. Using the assay of the present invention 100% of the tumors with IDH1 and IDH2 mutations and 98% of the tumors bearing IDH1 and IDH2 wild type proteins could be correctly classified. In contrast, the IDH1-R132H antibody detects only the IDH1 protein with the R132H mutation. However, this mutation only occurs in about 93% of the mutant IDH1 proteins. Furthermore, the IDH1-R132H antibody does not detect mutant IDH2 proteins which occur in about 4% tumors with mutant IDH1 and/or IDH2 proteins. Therefore, the IDH1-R132H antibody will correctly classify only 89% of tumors harboring the mutant IDH1 and IDH2 proteins. Thus, the specificity of the immunohistochemical IDH1 and IDH2 status analysis is significantly increased from about 89% in known methods to 100% in the method of the present invention.

However, the method of the present invention is not restricted to diagnosis of tumors characterized by IDH1/2 mutations but generally allows to make a prognosis as regards the progression of a tumor based on the determination of the level of BCAT1. In other words, the assay of the present invention allows the fast and reliable diagnostic and prognostic classification of tumors based on the activity of branched chain amino acid metabolism as reflected by the level or activity of BCAT1.

Thus, the present invention relates to methods for the diagnosis of a brain tumor, and for the estimation of a prognosis for patients afflicted with such tumor based on the determination of expression of BCAT1 in a patient sample.

### DETAILED DESCRIPTION OF THE INVENTION

The present invention provides a diagnostic method for the estimation of a prognosis for patients afflicted with a tumor, comprising
(a) obtaining a tumor sample from a patient; and
(b) determining the concentration of the BCAT1 protein in the sample, preferably by use of an anti-BCAT1 antibody;
   whereby a patient showing an increased concentration of BCAT1 has a worse prognosis compared to a patient showing a normal concentration of BCAT1.

The term "increased concentration" as used in the present invention means that the concentration is increased at least by a factor of 3 as compared to a control sample, e.g. normal brain tissue found at the resection margin adjacent to the tumor.

The term "prognosis" concerns an estimation of the overall survival time in months.

The term "worse prognosis compared to a patient showing a normal concentration of BCAT1" as used herein means that the probability of having a given remaining expectancy of life is substantially decreased.

Determination of the level of the BCAT1 protein by immunological methods using an anti-BCAT1 antibody is preferred. However, the person skilled in the art knows further assays for determining the concentration of the protein, e.g., assays based on the determination of a biological activity of the protein as a branched chain aminotransferase.

According to the present invention said tumor is a brain tumor. However, a diagnostic value has also be seen for patients with acute myeloid leukemia (AML) and all other types of tumors carrying mutations of the IDH1 and/or IDH2 proteins, as well as for tumors with variable activity of branched chain amino acid metabolism.

The term "tumor sample" or "brain tumor sample" as used herein, refers to a sample obtained from a patient. The tumor sample can be obtained from the patient by routine measures known to the person skilled in the art, i.e., biopsy (taken by aspiration or punctuation, excision or by any other surgical method leading to biopsy or resected cellular material). For those areas not easily reached via an open biopsy, a surgeon can, through a small hole made in the skull, use stereotaxic instrumentation to obtain a "closed" biopsy. Stereotaxic instrumentation allows the surgeon to precisely position a biopsy probe in three-dimensional space to allow access almost anywhere in the brain. Therefore, it is possible to obtain tissue for the diagnostic method of the present invention.

The term "brain tumor" is not limited to any stage, grade, histomorphological feature, invasiveness, aggressiveness or malignancy of an affected tissue or cell aggregation. In particular grade I, grade II, grade III or grade IV brain tumors, and all other types of cancers, malignancies and transformations associated with the brain are included. A preferred brain tumor to be diagnosed by the method of the present invention is a glioma. Preferred are anaplastic astrocytomas, anaplastic oligoastrocytomas and anaplastic oligodendrogliomas, in particular fibrillary astrocytoma WHO grade II, oligoastrocytoma WHO grade II, oligodendroglioma grade II, anaplastic astrocytoma WHO grade III, anaplastic oligoastrocytoma WHO grade III, anaplastic oligodendroglioma grade III or glioblastoma.

The term "antibody" as used herein relates to any type of antibody known in the art. An antibody as used herein includes intact immunoglobulin molecules, as well as fragments thereof, such as Fab, F(ab)₂, and Fv, which are capable of binding an epitope of BCAT1. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to the BCAT1 protein can be used in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

An antibody useful in the diagnostic method of the present invention can be raised according to well established methods, i.e., an BCAT1 polypeptide (Schuldiner O, Eden A, Ben-Yosef T, Yanuka O, Simchen G, Benvenisty N. Proc Natl Acad Sci USA. 1996 Jul 9;93(14):7143-8) can be used to immunize a mammal, such as a mouse, rat, rabbit, guinea pig, monkey, or human, to produce polyclonal antibodies. If desired, the (poly)peptide used as an immunogen can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to BCAT1 can be prepared using any technique which provides for the production of antibody molecules by continuous cell lines in culture. These techniques include, but are not limited to, the hybridoma technique, the human B cell hybridoma technique, and the EBV hybridoma technique [Kohler et al., Nature 256 (1985), 495-7). Moreover, a monoclonal anti-BCAT1 antibody is commercially available (BD Biosciences, San Jose, CA, USA).

Techniques described for the production of single chain antibodies can be adapted using methods known in the art to produce single chain antibodies which specifically bind to the BCAT1 protein. Antibodies with related specificity, but of distinct idiotypic composition, can be generated by chain shuffling from random combinatorial immunoglobulin libraries [Burton, PNAS USA 88 (1991), 11120-3). Single-chain antibodies also can be constructed using a DNA amplification method, such as PCR, using hybridoma cDNA as a template [Thirion et al., Eur. J. Cancer Prev. 5 (1996), 507-11). Single-chain antibodies can be mono- or bispecific, and can be bivalent or tetravalent. Construction of tetravalent, bispecific single-chain antibodies is taught, for example, in Coloma & Morrison, Nat. Biotechnol. 15 (1997), 159-63). Construction of bivalent, bispecific single-chain antibodies is taught in Mallender & Voss, J.Biol.Chem. Xno9 (1994), 199-206).

A nucleotide sequence encoding a single-chain antibody can be constructed using manual or automated nucleotide synthesis, cloned into an expression construct using standard recombinant DNA methods, and introduced into a cell to express the coding sequence. Alternatively, single-chain antibodies can be produced directly using, for example, filamentous phage technology (Verhaar et al., Int. J. Cancer 61 (1995), 497-501).

Antibodies useful in a method of the invention can be purified by methods well known in the art. For example, antibodies can be affinity purified by protein-A protein-G column chromatography. The bound antibodies can then be eluted from the column using a buffer with a high salt concentration.

The invention is not limited to a particular immunoassay procedure, and therefore is intended to include both homogeneous and heterogeneous procedures. Exemplary immunoassays which can be conducted according to the invention include fluorescence polarisation immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

The present invention also provides a method for distinguishing between (a) a brain tumor characterized by an IDH1-and/or IDH2 protein with a mutation and (b) a brain tumor characterized by a IDH1- and/or IDH2 protein without a mutation, comprising
(a) obtaining a brain tumor sample from a patient; and
(b) indirectly determining the presence of a mutation by determining the concentration of the BCAT1 protein in the sample, preferably by use of an anti-BCAT1 antibody;
   whereby a non-increased concentration of BCAT1 is indicative of a tumor characterized by an IDH1- and/or IDH2 protein with a mutation.

In this context, the term "mutation" means any gain of function mutation that alters the enzymatic reaction leading to the generation of 2-hydroxyglutarate as one of the end products. Examples of such mutations comprise amino acid substitutions affecting amino acid residue 132 in the IDH1 protein including but not limited to the substitutions R132H, R132C, R132S, R132G, and R132L and amino acid substitutions affecting amino acid residue 172 in the IDH2 protein including, but not limited to the substitutions R172K, R172M, and R172W.

The term "decreased concentration of BCAT1" includes substantial absence of BCAT1 (as determined by immunoassays, e.g. immunohistochemistry.)

The method of the present invention described in more detail in the examples overcomes the problems discussed above since it allows an indirect, fast, simple and reliable analysis of the IDH1/2 status by immunohistochemistry.

Preferably, the mutation indirectly detected by use of the method of the invention is a mutation within the IDH1 protein leading to better prognosis. Particularly preferred is a mutation at position R132 of the amino acid sequence of IDH1, e.g., R132H, R132C and R132S. Alternatively, the mutation indirectly detected by use of the method of the invention is a mutation within the IDH2 protein leading to better prognosis. Particularly preferred is a mutation at position R172 of the amino acid sequence of IDH2, e.g., R172K. Finally, the present invention also provides a method of selecting a therapy modality for a patient afflicted with a brain tumor, comprising
(a) obtaining a brain tumor sample from a patient; and
(b) determining the concentration of the BCAT1 protein in the sample, preferably by use of an anti-BCAT1 antibody; whereby the selection of a therapy modality depends on the concentration of BCAT1.

The terms "therapy modality" or "mode of treatment" refer to a timely sequential or simultaneous administration of antitumor, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, timeframe of application and frequency of administration within a defined therapy window.

Thus, in a preferred embodiment of the method of the present invention the mode of treatment to be chosen acts on cell proliferation, cell survival, cell motility, and/or angiogenesis.

In a more preferred embodiment, the mode of treatment comprises chemotherapy, administration of small molecule inhibitors, antibody based regimen, anti-proliferation regimen, pro-apoptotic regimen, pro-differentiation regimen, radiation and/or surgical therapy.

The determination of the level of BCAT1 has an important influence on the therapeutic procedure. Currently, anaplastic astrocytomas WHO grade III are separated from glioblastomas WHO grade IV by the presence or absence of necrosis or vascular proliferation. However, the results of the NOA-04 study (Randomized phase III trial of sequential radiochemotherapy of anaplastic glioma with procarbazine, lomustine, and vincristine or temozolomide) show that the prognosis of (histologically defined) anaplastic astrocytomas WHO grade III without IDH1 mutations is more or less identical to the prognosis of glioblastomas WHO grade IV.

In the therapy of malignant gliomas different protocols are applied to anaplastic astrocytomas WHO grade III and glioblastomas WHO grade IV. Later tumors are treated much more radical by combined radio- and chemotherapy whereas patients with anaplastic astrocytomas WHO grade III receive such bimodal therapy only if they are younger than 40 years of age. The IDH1 status allows the identification of those anaplastic astrocytomas WHO grade III that have a prognosis similar to glioblastomas WHO grade IV and that should be treated like such highest malignant brain tumors.

### BRIEF DESCRIPTION OF THE DRAWINGS

Figure 1: BCAT1-protein expression in astrocytic gliomas Tumor sections on a tissue microarray were stained using the antibody against the BCAT1 protein. Representative examples of BCAT1-positive (A) and BCAT1-negative (B) tumors are shown.
Figure 2: Kaplan-Meier plot of BCAT1 protein expression and overall survival

The following examples illustrate the invention, but are not to be construed as limitations of the invention.

### Example 1

### Materials and Methods

### (A) Patients

Tumor tissues of astrocytic gliomas were selected from the collections at the Department of Neuropathology, Heinrich-Heine-University Düsseldorf, Germany. All tumors were histologically classified according to the criteria of the WHO 2000 classification of tumors of the nervous system, which in case of astrocytic gliomas have been retained in the revised WHO classification of 2007.1. Clinical samples were obtained after informed consent and follow-up data of the patients retrospectively determined and linked to the molecular data in an anonymized manner as approved by the Institutional Review Board of the Medical Faculty, Heinrich-Heine-University, Düsseldorf.

Paraffin blocks from some tumors were available for the construction of tissue micro-arrays (TMAs). HE (hematoxylin and eosin) stained sections from the donor blocks were used to define representative tumor regions. Up to three different tissue cylinders with a diameter of 0.8 mm were taken from each donor block from selected areas using a tissue chip microarrayer (Beecher Instruments, Sun Prairie, WI, USA) and transferred to a recipient paraffin block. The recipient paraffin block was cut in 5 µm paraffin sections using a Leica DSC1 microtome (Leica Microsystems, Wetzlar, Germany).

### (B) Immunohistochemical detection of BCAT1 protein

Tumor tissue sections and tumor tissue microarrays were deparaffinized using xylol and rehydrated with graded ethanol. Antigen retrieval was performed by heating for 40 min in a steamer in 10 mM sodium citrate buffer (pH 6.0). Endogenous peroxidase was inactivated by incubating the tissues in 3% hydrogen peroxide. TMAs were incubated overnight with primary anti-ECA39 (BCAT1) monoclonal antibody, clone 51 (BD Biosciences, San Jose, CA) diluted 1:3000 in Dako REAL™ antibody diluent (Dako, Glostrup, Denmark). Staining for detection of bound antibody was performed according to standard protocols using the DakoREAL™ Detection System (Peroxidase/DAB+, rabbit/mouse) (Dako, Glostrup, Denmark), subsequent counterstaining was done using hematoxylin. Tumors were scored as either positive or negative depending on the intensity of BCAT1 staining. Evaluation of immunohistochemical staining was carried out blinded from clinical data.

### (C) Mutation analyses

The IDH1 and IDH2 genes were investigated for mutations using the primers IDH1-sense 5' -accaaatggcaccatacgaa -3' and IDH1-antisense 5'- acatgcaaaatcacattattgcc -3' that amplify a 168-bp IDH1-fragment spanning codon 132 or IDH2-sense 5'-ccaatggaactatccggaac -3' and IDH2-antisense 5'-tgtggccttgtactgcagag -3'amplifying a 227-bp IDH2-fragment including codon 172, respectively. The PCR products were purified and sequenced in both directions using cycle sequencing and an ABI PRISM 377 semi-automated DNA sequencer (Applied Biosystems, Foster City, CA).

### Example 2

### Analysis of BCAT1 protein expression in different tumor samples by immunohistochemistry

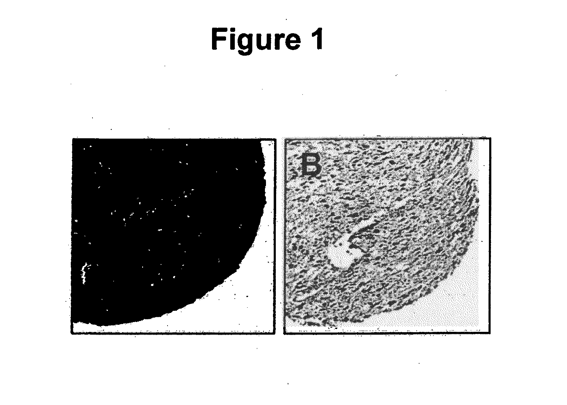

BCAT1 protein expression was analyzed by immunohistochemistry in 81 glioma samples, including 55 glioblastomas of WHO grade IV, 12 anaplastic astrocytomas of WHO grade III (AAIII), 3 anaplastic oligodendrogliomas WHO grade III (AOIII), 10 diffuse astrocytomas of WHO grade II (AII), and 1 oligodendroglioma of WHO grade II. The glioblastoma group included 51 primary glioblastomas (pGBIV) and 4 secondary glioblastomas (sGBIV). 32 of the tumors were stained as whole-tumor sections and 39 as part of a tissue microarray. BCAT1 stainings were scored as either positive or negative. Typical staining patterns representing these two categories are shown in Figure 1.

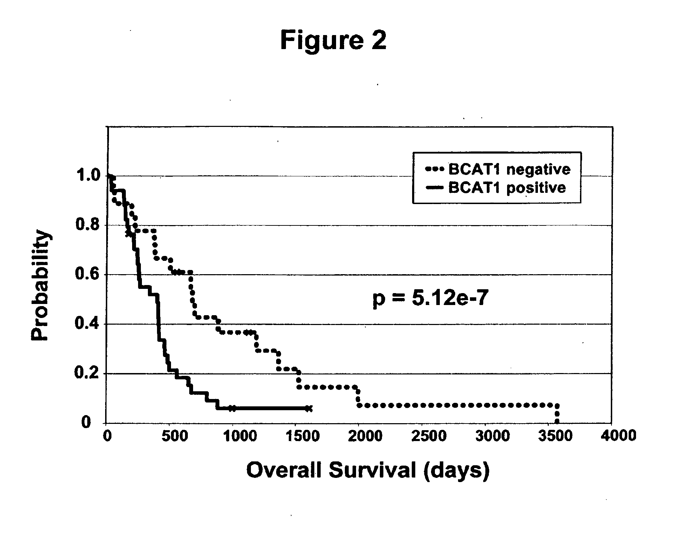

Correlation of BCAT1 protein expression and overall survival was possible for 67 tumor samples for which survival data was available. Of these tumors 43 showed high BCAT1 protein expression and 24 showed low BCAT1 protein expression. Survival analysis by the method of Kaplan and Meier indicated a highly significant (p=5.12e-7) correlation of high BCAT1 protein expression with adverse prognosis (Figure 2).

BCAT1 protein expression also was correlated with the mutation status of the IDH1 and IDH2 genes. For this purpose the mutation status of the IDH1 and IDH2 genes first was determined by sequencing the region including codon 132 of the IDH1 gene and codon 172 of the IDH2 gene. Mutations of the IDH1 gene were found in 32 tumor samples, including 30 R132H, 1 R132C and 1 R132S mutations. Mutations of the IDH2 gene were present in 3 tumors (all R172K). 45 tumors harboured neither IDH1 nor IDH2 mutations. Analysis of these data using the Fisher's Exact test revealed a highly significant inverse correlation of BCAT1 expression and mutation of the IDH1 or IDH2 genes (p=5.23e-22; Table 1). Of note, the BCAT1 did not label any of the tumors harbouring any type of IDH1 or IDH2 mutant proteins.

**Table 1**

| **Fisher's Exact Test shows a strong inverse correlation of BCAT1 protein expression and mutation status of the IDH1 and IDH2 genes (p=3.53e-22)** | | |
|---|---|---|
| | BCAT1 positive tumors | BCAT1 negative tumors |
| Tumors with IDH1 and IDH2 wild type genes | 45 | 1 |
| Tumors with IDH1 or IDH2 mutant genes | 0 | 35 |

These data show that BCAT1 protein expression, in addition to being a prognostic marker in gliomas (Figure 1), also is a diagnostic marker that, due to its high specificity and sensitivity, is excellently suited for classifying tumors with and without mutations of the IDH1 and IDH2 genes. To diagnose these prognostically important tumor groups, an antibody specific for the IDH1-R132H mutant protein currently is used. However, since R132H mutations constitute only 93% of the number of IDH1 mutantions and only 89% of the combined number of IDH1 and IDH2 mutations, this antibody will incorrectly classify a significant proportion of gliomas.

## Claims

1. A diagnostic method for the estimation of a prognosis for patients afflicted with a brain tumor, comprising
determining, in a brain tumor sample from a patient, the concentration of the BCAT1 protein in the sample;
whereby a patient showing an increased concentration of BCAT1 has a worse prognosis compared to a patient showing a normal concentration of BCAT1.

2. The method claim 1, wherein said brain tumor is a glioma.

3. The method of claim 2, wherein said glioma is glioblastoma grade IV, anaplastic astrocytoma grade III, anaplastic oligodendroglioma grade III, diffuse astrocytoma grade II, or oligodendroglioma grade II.

4. The method of claim 2 wherein the tumor is a low grade astrocytoma or an oligodendroglioma.

5. A method for distinguishing between (a) a brain tumor **characterized by** an IDH1- and/or IDH2 protein with a mutation and (b) a brain tumor **characterized by** an IDH1- and/or IDH2 protein without a mutation, comprising
indirectly determining, in a brain tumor sample from a patient; the presence of a mutation by determining the concentration of the BCAT1 protein in the sample;
whereby a non-increased concentration of BCAT1 is indicative of a tumor **characterized by** an IDH1- and/or IDH2 protein with a mutation.

6. The method claim 5, wherein said brain tumor is a glioma.

7. The method of claim 5, wherein said mutation is a mutation of the IDH1 protein.

8. The method of claim 7, wherein said mutation is at position R132 of the amino acid sequence.

9. The method of claim 8, wherein said mutation is R132H.

10. The method of claim 5, wherein said mutation is a mutation of the IDH2 protein.

11. The method of claim 10, wherein said mutation is at position R172 of the amino acid sequence.

12. The method of claim 11, wherein said mutation is R172K.

13. A method of selecting a therapy modality for a patient afflicted with a brain tumor, comprising
determining in a brain tumor sample from a patient; the concentration of the BCAT1 protein in the sample;
whereby the selection of a therapy modality depends on the concentration of BCAT1.

14. The method claim 13, wherein said brain tumor is a glioma.

15. The method of claim 13, wherein said therapy modality acts on cell proliferation, cell survival cell motility, and/or angioneogenesis, preferably wherein said therapy modality comprises chemotherapy, administration of a small molecule inhibitor, antibody based regimen, anti-proliferation regimen, pro-apoptotic regimen, pro-differentiation regimen, radiation and/or surgical therapy.

## Patentansprüche

1. Diagnostisches Verfahren zur Einschätzung einer Prognose für Patienten, die an einem Hirntumor leiden, umfassend
Bestimmen in einer Hirntumorprobe eines Patienten die Konzentration des BCAT1-Proteins in der Probe;
wobei ein Patient, der eine erhöhte Konzentration an BCAT1 zeigt, eine schlechtere Prognose im Vergleich zu einem Patienten hat, der eine normale BCAT1-Konzentration zeigt.

2. Verfahren nach Anspruch 1, wobei der Hirntumor ein Gliom ist.

3. Verfahren nach Anspruch 2, wobei das Gliom ein Glioblastom der Stufe IV, ein anaplastisches Astrozytom der Stufe III, ein anaplastisches Oligodendrogliom der Stufe III, ein diffuses Astrozytom der Stufe II oder ein Oligodendrogliom der Stufe II ist.

4. Verfahren nach Anspruch 2, wobei der Tumor ein niedrigstufiges Astrozytom oder ein Oligodendrogliom ist.

5. Verfahren zum Unterscheiden zwischen (a) einem Hirntumor, der durch ein IDH1- und/oder IDH2-Protein mit einer Mutation gekennzeichnet ist, und (b) einem Hirntumor, der durch ein IDH1- und/oder IDH2-Protein ohne eine Mutation gekennzeichnet ist, umfassend
indirektes Bestimmen in einer Hirntumorprobe eines Patienten, ob eine Mutation vorliegt, indem die Konzentration des BCAT1-Proteins in der Probe bestimmt wird;
wobei eine nicht erhöhte BCAT1-Konzentration ein Anzeichen für einen Tumor ist, der durch ein IDH1- und/oder IDH2-Protein mit einer Mutation gekennzeichnet ist.

6. Verfahren nach Anspruch 5, wobei der Hirntumor ein Gliom ist.

7. Verfahren nach Anspruch 5, wobei die Mutation eine Mutation des IDH1-Proteins ist.

8. Verfahren nach Anspruch 7, wobei sich die Mutation in Position R 132 der Aminosäuresequenz befindet.

9. Verfahren nach Anspruch 8, wobei die Mutation R 132H ist.

10. Verfahren nach Anspruch 5, wobei die Mutation eine Mutation des IDH2-Proteins ist.

11. Verfahren nach Anspruch 10, wobei sich die Mutation in der Position R 172 der Aminosäuresequenz befindet.

12. Verfahren nach Anspruch 11, wobei die Mutation R172K ist.

13. Verfahren zum Auswählen einer Therapiemodalität für einen Patienten, der an einem Hirntumor leidet, umfassend
Bestimmen in einer Hirntumorprobe eines Patienten die Konzentration an BCAT1-Protein in der Probe;
wobei das Auswählen einer Therapiemodalität von der BCAT1-Konzentration abhängt.

14. Verfahren nach Anspruch 13, wobei der Hirntumor ein Gliom ist.

15. Verfahren nach Anspruch 13, wobei die Therapiemodalität auf die Zellproliferation, das Zellüberleben, die Zelibeweglichkeit und/oder die Angioneogenese einwirkt, wobei vorzugsweise die Therapiemodalität eine Chemotherapie, die Verabreichung eines niedermolekularen Inhibitors, ein auf Antikörpern basierender Therapieplan, ein Antiproliferations-Therapieplan, ein pro-apopotischer Therapieplan, ein Pro-Differenzierungs-Therapieplan, eine Bestrahlung und/oder eine operative Therapie umfasst.

## Revendications

1. Méthode de diagnostic pour estimer un pronostic pour des patients atteints d'une tumeur cérébrale, comprenant le fait de
déterminer, dans un échantillon de tumeur cérébrale d'un patient, la concentration de protéine BCAT1 dans l'échantillon; un patient présentant une concentration augmentée de BCAT1 présentant un pire pronostic par rapport à un patient présentant une concentration normale de BCAT1.

2. Méthode selon la revendication 1, dans laquelle ladite tumeur cérébrale est un gliome.

3. Méthode selon la revendication 2, dans laquelle ledit gliome est le glioblastome de grade IV, l'astrocytome anaplasique de grade III, l'oligodendrogliome anaplasique de grade III, l'astrocytome diffus de grade II ou l'oligodendrogliome de grade II.

4. Méthode selon la revendication 2, dans laquelle la tumeur est un astrocytome ou un oligodendrogliome de bas grade.

5. Méthode pour distinguer entre (a) une tumeur cérébrale **caractérisée par** une protéine IDH1 et/ou IDH2 avec mutation et (b) une tumeur cérébrale **caractérisée par** une protéine IDH1 et/ou IDH2 sans mutation, comprenant le fait de
déterminer indirectement, dans un échantillon de tumeur cérébrale d'un patient, la présence d'une mutation par détermination de la concentration de la protéine BCAT1 dans l'échantillon;
une concentration non accrue de BCAT1 indiquant une tumeur **caractérisée par** une protéine IDH1 et/ou IDH2 avec mutation.

6. Méthode selon la revendication 5, dans laquelle ladite tumeur cérébrale est un gliome.

7. Méthode selon la revendication 5, dans laquelle ladite mutation est une mutation de la protéine IDH 1.

8. Méthode selon la revendication 7, dans laquelle ladite mutation se produit à la position R132 de la séquence d'acides aminés.

9. Méthode selon la revendication 8, dans laquelle ladite mutation est R132H.

10. Méthode selon la revendication 5, dans laquelle ladite mutation est une mutation de la protéine IDH2.

11. Méthode selon la revendication 10, dans laquelle ladite mutation se produit à la position R172 de la séquence d'acides aminés.

12. Méthode selon la revendication 11, dans laquelle ladite mutation est R172K.

13. Méthode pour sélectionner une modalité de thérapie pour un patient atteint d'une tumeur cérébrale, comprenant le fait de
déterminer, dans un échantillon de tumeur cérébrale d'un patient, la concentration de la protéine BCAT1 dans l'échantillon; la sélection d'une modalité de thérapie dépendant de la concentration de BCAT1.

14. Méthode selon la revendication 13, dans laquelle ladite tumeur cérébrale est un gliome.

15. Méthode selon la revendication 13, dans laquelle ladite modalité de thérapie agit sur la prolifération cellulaire, la survie cellulaire, la motilité cellulaire et/ou l'angionéogenèse, de préférence dans laquelle ladite modalité de thérapie comprend la chimiothérapie, l'administration d'un inhibiteur de petites molécules, un traitement à base d'anticorps, un traitement anti-prolifération, un traitement pro-apoptotique, un traitement pro-différenciation, une radiothérapie et/ ou une thérapie chirurgicale.
